# FASCICULE DE BREVET EUROPEEN

(11) **EP 1 960 548 B1**
(45) Date de publication et mention de la délivrance du brevet: **17.06.2009**
(21) Numéro de dépôt: 06841843.3
(22) Date de dépôt: 01.12.2006
(51) Int. Cl.: C12Q 1/68, C12Q 1/02

(54) **PROCEDE DE PREDICTION DE LA VIABILITE ET DE LA VITALITE DE BACTERIES DESTINEES A ETRE UTILISEES DANS DES ENVIRONNEMENTS STRESSANTS**
VERFAHREN ZUR VORHERSAGE DER LEBENSFÄHIGKEIT UND VITALITÄT VON UNTER STRESSBEDINGUNGEN EINSETZBAREN BAKTERIEN
METHOD FOR PREDICTING THE VIABILITY AND VITALITY OF BACTERIA USABLE IN STRESSING ENVIRONMENT

(30) Priorité: 02.12.2005 FR 0512292
(43) Date de publication de la demande: 27.08.2008
(73) Titulaire: Université de Bourgogne, 21078 Dijon (FR)
(72) Inventeur: GUZZO, Jean, F-21121 Fontaine les Dijon (FR); DESROCHE, Nicolas, F-21000 Dijon (FR); TOURDOT-MARECHAL, Raphaëlle, F-21000 Dijon (FR)
(74) Mandataire: Oudin, Stéphane
(86) Numéro de dépôt international: PCT/FR2006/002633
(87) Numéro de publication internationale: WO 2007/063228

(56) Documents cités:
- WO-A-97/17463
- US-A- 4 380 552
- BELTRAMO ET AL: "Real-time PCR for characterizing the stress response of Oenococcus oeni in a wine-like medium" RESEARCH IN MICROBIOLOGY, ELSEVIER, AMSTERDAM, NL, vol. 157, no. 3, 2 septembre 2005 (2005-09-02), pages 267-274, XP005342853 ISSN: 0923-2508 cité dans la demande
- GUZZO J ET AL: "Regulation of stress response in Oenococcus oeni as a function of environmental changes and growth phase." INTERNATIONAL JOURNAL OF FOOD MICROBIOLOGY. 10 APR 2000, vol. 55, no. 1-3, 10 avril 2000 (2000-04-10), pages 27-31, XP008065631 ISSN: 0168-1605
- SANDERS J W ET AL: "Environmental stress responses in Lactococcus lactis" FEMS MICROBIOLOGY REVIEWS, ELSEVIER, AMSTERDAM, NL, vol. 23, no. 4, juillet 1999 (1999-07), pages 483-501, XP002257074 ISSN: 0168-6445
- LIU S-Q ET AL: "GROWTH AND METABOLISM OF SELECTED LACTIC ACID BACTERIA IN SYNTHETIC WINE" AMERICAN JOURNAL OF ENOLOGY AND VITICULTURE, vol. 46, no. 2, 1995, pages 166-174, XP009021611 ISSN: 0002-9254

## Description

La présente invention concerne un procédé de prédiction de la viabilité, de la vitalité et de la stabilité de bactéries destinées à être utilisées dans des environnements stressants ainsi que l'utilisation de ce procédé pour le contrôle qualité de l'adaptation au stress de préparations bactériennes constituées desdites bactéries et/ou pour la sélection de bactéries entrant dans la composition de telles préparations.

Dans le domaine de la vinification, on recourt à des ferments levuriens pour la fermentation alcoolique et à des ferments bactériens pour la fermentation malolactique.

A cette fin, il a été développé des préparations de ferments bactériens qui permettent le déclenchement de la fermentation malolactique au moment opportun du processus de vinification, par inoculation des ferments dans le vin. Cette inoculation, ou ensemencement, peut être réalisée de façon directe ou après réactivation des ferments. *Oenococcus oeni* est l'espèce bactérienne la plus fréquemment utilisée et la mieux adaptée pour réaliser cette fermentation.

Les fabricants de ces préparations bactériennes ainsi que leurs utilisateurs sont confrontés au problème de la survie des bactéries lors de l'inoculation dans le vin : en effet, les conditions physico-chimiques du vin sont extrêmement défavorables à l'implantation de ces ferments lactiques et causent donc une mortalité importante des bactéries. Afin de réduire ce problème et d'optimiser l'utilisation de ces ferments malolactiques, les producteurs de bactéries ont mis au point des protocoles de pré-acclimatation des bactéries. Les cultures bactériennes sont soumises à une série de stress physico-chimiques forçant les bactéries à adapter leur structure cellulaire et leur métabolisme, permettant d'obtenir des souches capables de survivre après ensemencement dans le vin.

D'une manière plus générale, les ferments bactériens, et en particulier les ferments lactiques, sont soumis à des conditions défavorables (acidité, carence nutritionnelle, stress oxydatif, etc.) lors de leur mise en oeuvre. Les producteurs de ces préparations bactériennes sont donc confrontés au problème de garantir une survie, une vitalité et une stabilité desdites préparations lors de leur stockage ou lors de leur utilisation dans des procédés fermentaires ou encore, dans le cas des probiotiques, lors de leur ingestion et notamment la traversée de l'estomac. Par probiotiques, on entend les suppléments alimentaires composés de bactéries vivantes qui exercent un effet bénéfique en améliorant l'équilibre de la flore intestinale ; ces ferments probiotiques contiennent des lactobacilles, des bifidobactéries et des streptocoques.

La difficulté aujourd'hui pour ces producteurs de préparations bactériennes est donc d'obtenir une mesure fiable du niveau d'adaptation aux stress des préparations bactériennes, pré-acclimatées ou non, et de leur aptitude à survivre après inoculation dans des milieux ; il leur importe également de valider la qualité des produits obtenus après congélation ou lyophilisation, leur permettant de proposer les bactéries les plus performantes dans les préparations commercialisées.

Dans le cas des ferments malolactiques, les industriels ont actuellement recours à des expériences de micro-vinification qui sont coûteuses, longues (plusieurs semaines) et dispendieuses en main d'oeuvre. De surcroît, elles sont éloignées des conditions réelles d'utilisation des ferments, à cause des faibles volumes considérés et de la difficulté d'utiliser dans ces expériences un vin représentatif des vins des utilisateurs finaux de ces ferments.

D'autres tests ont également été développés, basés sur l'influence des bactéries sur des paramètres physiques de leur environnement, comme la concentration de certains métabolites que ces bactéries consomment ou produisent.

On utilise également la technique conventionnelle de numération cellulaire, qui consiste à effectuer des prélèvements réguliers du milieu stressant dans lequel les bactéries ont été implantées, puis à mettre en culture ces prélèvements et à compter les bactéries après une période d'incubation définie. Or si cette technique permet d'observer la croissance de la population bactérienne soumise au stress, elle ne renseigne que partiellement sur l'état physiologique des bactéries : en effet, il est connu que des bactéries peuvent se multiplier en milieu de culture sans être pleinement fonctionnelles et inversement, des bactéries adaptées aux conditions de stress peuvent ne pas être aptes à se multiplier en milieu de culture. Cette technique ne permet donc pas de contrôler avec fiabilité et reproductibilité la capacité des bactéries à survivre en environnement stressant, suite à leur préparation industrielle.

WO 97/17463 A décrit un procédé de prédiction de la viabilité, vitalité et stabilité de bactéries destinées à être utilisées dans des environnements stressants, comprenant la détermination de la proportion de bactéries vivantes, mortes ou stressées dans une culture comme indicateur quantitatif de la capacité de la population bactérienne à résister à un stress de long terme (résumé; p. 4 l. 1-14; p. 4-6; revendication 1; revendication 17). La détermination de la proportion de bactéries vivantes, mortes ou stressées est effectuée à l'aide de marqueurs fluorescents ayant la capacité de marquer soit toutes les cellules, soit les cellules ayant une membrane endommagée (p. 6 I. 9-11).

Aucun de ces tests n'est aujourd'hui réellement satisfaisant : d'une part, ils sont fastidieux et réclament une durée de réalisation importante, et d'autre part, leur degré de précision ne donne pas entière satisfaction en terme d'outils de contrôle qualité.

La présente invention a donc pour but de circonvenir aux difficultés sus-mentionnées en proposant un procédé fiable, reproductible, précis et rapide qui permet d'une part, d'estimer et de valider le niveau d'adaptation aux stress de préparations bactériennes produites industriellement et destinées à être utilisées dans des milieux hostiles à la survie de ces bactéries et d'autre part, de sélectionner des souches bactériennes constitutives de telles préparations.

A cet égard, la présente invention a pour objet un procédé de prédiction de la viabilité, de la vitalité et de la stabilité de bactéries destinées à être utilisées dans des environnements stressants, lesquelles bactéries sont produites sous forme de préparations bactériennes et conditionnées sous forme congelée ou lyophilisée, remarquable en ce que ledit procédé comporte :
- une étape préalable de remise en suspension desdites préparations bactériennes dans un milieu nutritif aqueux non stressant ;
- une mesure moléculaire du niveau d'expression d'au moins un gène marqueur représentatif de l'état de stress pour chaque espèce bactérienne considérée ;
- une mesure du pH intracellulaire des bactéries basée sur une sonde fluorescente sensible au pH ;
- une étape de comparaison des valeurs mesurées à celles de témoins et/ou d'étalons de référence.

Par milieu nutritif aqueux, on entend un milieu contenant des substances utiles à l'activité normale des bactéries et à leur survie, sans pour autant constituer un milieu de croissance qui permettrait à ces bactéries de se multiplier ; en outre, ce milieu est non stressant, c'est-à-dire qu'il constitue un environnement neutre sur le plan physico-chimique, permettant une activité énergétique normale des bactéries. Par exemple, un tel milieu est l'eau peptonée (NaCl à 9g.L⁻¹ et peptone à 1g.L⁻¹), qui est classiquement utilisée en microbiologie pour les opérations de dilution.

La dernière étape du procédé selon l'invention permet de définir précisément les caractéristiques de vitalité, de viabilité et de stabilité des bactéries considérées lorsqu'elles seront ultérieurement placées dans un environnement stressant.

La présente invention a également pour objet l'utilisation de ce procédé pour le contrôle de l'adaptation aux stress de ferments bactériens. Ces ferments peuvent être obtenus par des procédés de pré-acclimatation ou par présélections de souches bactériennes. L'adaptation au stress acide est plus particulièrement concernée. Une des utilisations particulièrement privilégiée concerne le contrôle des ferments malolactiques destinés à l'ensemencement du vin.

Selon un premier aspect de l'invention, on procède à une mesure moléculaire qui est basée sur la mesure du niveau d'expression d'au moins un gène marqueur représentatif de l'état de stress des espèces bactériennes considérées.

Selon un second aspect de l'invention, on mesure le pH interne, c'est-à-dire une mesure de l'état physiologique qui traduit la viabilité et la vitalité des bactéries et qui est liée à l'intégrité membranaire des bactéries.

La caractérisation de la stabilité physiologique dans le temps de ces bactéries est réalisée en répétant les deux types de mesures sur une période de temps correspondante à la durée de stockage et/ou d'utilisation des bactéries.

On comprend bien que la combinaison de ces deux paramètres permet une interprétation fiable de la qualité de survie et d'activité des bactéries après inoculation : on vérifie, par la quantification de l'expression génique, l'adaptation aux conditions de stress des bactéries, et on s'assure, par la caractérisation de l'état physiologique, que ces bactéries sont viables et présentent un niveau de vitalité satisfaisant, la mise en corrélation de ces données conduisant à une information complète et significative de l'adaptation des bactéries.

On compare les valeurs mesurées à celles de témoins et/ou d'étalons pour définir le degré de l'expression génique et la nature de l'état physiologique afin de caractériser les bactéries testées. Les témoins seront généralement les mêmes souches bactériennes, n'ayant pas subi de pré-acclimatation à l'environnement stressant.

D'autres avantages et caractéristiques de l'invention ressortiront mieux de la description détaillée qui va suivre du procédé selon l'invention.

Selon une caractéristique essentielle de l'invention, le procédé met en oeuvre une mesure du niveau d'expression d'au moins un gène marqueur représentatif de l'état de stress des bactéries.

Ce niveau d'expression est quantifié par la technique de transcription inverse couplée à la PCR quantitative en temps réel (RT-qPCR).

De préférence, la mesure de la réponse au stress portera sur l'expression de plusieurs gènes marqueurs, ce qui augmente la fiabilité de la mesure. On choisira avantageusement des gènes qui s'expriment à différents stades de croissance des bactéries. De préférence, on choisira des gènes pour lesquels le rapport des niveaux d'expression entre population adaptée au stress et population non adaptée est d'au moins un facteur 2.

Ainsi, dans le cas des ferments malolactiques et de la bactérie *Oenococcus oeni*, l'expression du gène *hsp18* sera au minimum celle mesurée dans le cadre du procédé. Ce gène code la protéine chaperon small *Hsp Lo18*.

De préférence, on ajoutera la mesure de l'expression d'au moins un des gènes pris parmi *clpX, trxA* ou *cfa*, qui sont impliqués dans l'adaptation de *Oenvcoccus oeni* aux stress. L'expression de *hsp18* est fortement induite par de multiples stress, ainsi qu'en phase stationnaire. *ClpX* en revanche se distingue comme marqueur de la phase de latence tandis que *trxA* et *cfa* sont des marqueurs de stress exprimés pendant toutes les phases.

L'Homme de l'Art se référera aux bases de données génétiques publiant le génome de *Oenococcus oeni* pour déterminer les séquences des sondes nucléotidiques qui seront employées lors de la RT-qPCR. On se référera avantageusement à la publication BELTRAMO et al. Real-time PCR for characterizing the stress response of *Oenococcus oeni* in a wine-like medium. Research in microbiology, vol. 157, no.3, 02 septembre 2005, pages 267-274, qui présente des sequences possibles des amorces nucléotidiques.

On se référera également aux publications suivantes, pour les caractéristiques de ces gènes marqueurs pour *Oenococcus oeni* :
- Jobin, M.P., Delmas, F., Garmyn, D., Diviès C. and Guzzo, J. Molecular characterization of the gene encoding a 18-kDa small heat shock protein associated with the membrane of Leuconostos oenos. Appl. Env. Microbiol. 63 : 6309-614 (1997).
- Jobin, M.P., Garmyn, D., Diviès, C. and Guzzo, J. Expression of the Oenococcus oeni trxA gene is induced by hydrogen peroxide and heat shock. Microbiology. 145 : 1245-1251 (1999).
- Jobin, M.P., Garmyn, D., Diviès, C. and Guzzo, J. The Oenococcus oeni clpX homologue is a heat shock gene preferentially expressed in exponential growth phase. J. Bacteriol. 181 : 6634-6641 (1999).

Dans le cas de *Lactobacillus plantazum*, qui est un probiotique, on pourra avantageusement utiliser au minimum la mesure de l'expression du gène *hsp18.55*. De préférence, pour une meilleure pertinence des résultats, on ajoutera la mesure de l'expression d'au moins un des gènes pris parmi *hsp19.5, groEL ou cfa*.

Bien entendu, dans le cas d'autres préparations bactériennes, et en particulier les probiotiques, l'Homme de l'art choisira les marqueurs géniques les plus appropriés. De préférence, on ciblera des gènes codant pour des protéines de faible masse moléculaire induites lors de stress et pour lesquelles le rapport des niveaux d'expression entre population adaptée au stress et population non adaptée est d'au moins un facteur 2. En effet, de tels gènes, qui correspondent par exemple à *hsp18* chez *O. oeni* ou à *hsp18.55* ou *hsp19.5* chez *L*. *plantarum*, sont également présents chez les genres bactériens habituellement utilisés comme probiotiques (lactobacilles, bifidobactéries).

Le protocole de la mesure moléculaire est basé sur l'application conventionnelle de la technique de RT-qPCR et se décompose selon les principales étapes suivantes :
- première étape de remise en suspension des préparations bactériennes lyophilisées ou congelées, par exemple par réhydratation pendant quinze minutes à 30°C dans de l'eau peptonée (NaCl à 9g.L⁻¹ et peptone à 1g.L⁻¹) si les préparations sont lyophilisées;
- extraction des ARN et dosages des ARN par mesure spectrophotométrique à 260/280 nm ;
- traitement à la DNase et vérification de l'efficacité du traitement par PCR ;
- transcription inverse;
- PCR quantitative avec amorces spécifiques des gènes ciblés et utilisation d'un témoin interne pour la normalisation des résultats.

On utilise ensuite un étalonnage et/ou un comparatif avec des souches témoins pour qualifier l'adaptation aux stress des bactéries en fonction de l'expression génique mesurée.

On a ainsi obtenu dans le cas de souches d'Oenococcus oeni, des rapports de mesures des taux de transcrit entre ceux des cellules acclimatées et ceux des mêmes souches non acclimatées de 5 pour *hsp18*, de 2,25 pour *clpx*, de 5,5 pour *trxA*, et de 4 pour *cfa*. Les souches ont été acclimatées en les soumettant à un processus industriel de préacclimatation appliqué par les fabricants de ferments malolactiques.

Dans le cas de souches de *Lactobacillus plantarum*, le rapport des taux de transcrit entre les cellules acclimatées par cultures à pH acide et cellules non acclimatées est de 2,1 pour *hsp18.55*, 3,4 pour *hsp19.5*, 2,8 pour *groEL*, et 2,6 pour *cfa*.

Selon une autre caractéristique essentielle de l'invention, les procédés mettent en oeuvre une mesure de l'état physiologique qui traduit à la fois la viabilité et la vitalité des bactéries, qui est basée sur une mesure du pH intracellulaire (pHi)des bactéries. En effet, non seulement cette technique nous renseigne sur l'intégrité membranaire mais elle est également significative de la capacité à survivre en milieu acide, paramètre primordial dans le cas des ferments lactiques notamment.

De fait, les bactéries qui se sont bien adaptées aux conditions de stress ont un pHi significativement différent des témoins non adaptés. On peut donc déterminer, pour chaque espèce bactérienne et pour un milieu donné, des valeurs seuils et/ou des plages des valeurs de pHi avec lesquelles on caractérise l'état physiologique global des bactéries. Ainsi, dans le cas de souches d'*Oenococcus oeni* non acclimatées et acclimatées, on a déterminé pour une valeur de pH du milieu externe de 3,5, que le pHi des cellules non acclimatées est de 5,21 ± 0,15 alors que celui des cellules acclimatées est de 4,99 ± 0,05.

Dans le cas de *Lactobacillus plantarum*, le pHi pour un pH externe à 3,5 des cellules non acclimatées est de 5,41 t 0,06 tandis que celui des cellules acclimatées est de 5,02 ± 0,06.

Le protocole mis en oeuvre pourra être basé sur une mesure de pH intracellulaire via une sonde fluorescente sensible au pH, par exemple la carboxy-fluoresceine diacetate (CFDA). Ce protocole dérive de la technique décrite par Breeuwer *et al*. (Applied and Environmental Microbiology, Jan. 1996).

Le protocole appliqué pour *Oenococcus oeni* se décompose selon les étapes suivantes :
- première étape de remise en suspension des préparations bactériennes lyophilisées ou congelées dans un milieu nutritif non stressant ; par exemple, pour *Oenococcus oeni* on réhydratera les préparations lyophilisées pendant 15 minutes à 30°C dans de l'eau peptonée (NaCl : 9g.L⁻¹ et peptone : 1gL⁻¹) ;
- lavage des cellules dans un tampon Phosphate de Potassium 100 mM, pH 5,3 ;
- resuspension des cellules dans du tampon Phosphate de Potassium 100 mM, pH 8,0 avec la sonde CDCFDase à 1 µM et incubation 30 minutes à 30°C ; pendant cette étape on procède au chargement et à la conjugaison de la sonde ;
- centrifugation et resuspension des cellules dans du tampon Phosphate de Potassium 100 mM, pH 5,3 avec du glucose à 20 mM et incubation 30 minutes à 30°C ; on procède à l'élimination de la sonde non conjuguée ;
- centrifugation et resuspension des cellules dans du tampon Phosphate de Potassium 100 mM, pH 5,3, afin de procéder à l'élimination de la sonde non conjuguée présente dans le surnageant ;
- centrifugation et resuspension dans 2 mL de Tampon Citrate/Phosphate 100 mM à différents pH. Ajout de 1 µL de nigéricine et 1 µL de valinomycine pour calibration de la sonde ; cette étape a pour but la perméabilisation de la membrane aux protons ;
- lecture de la fluorescence émise à 531 nm après excitation à 440 nm (émission insensible au pH) et à 512 nm (émission sensible au pH) sur la suspension non filtrée (fluorescence totale) et sur la suspension filtrée sur filtre 0,22 µm (fluorescence extracellulaire) ;
- calcul du ratio = (Emission après Ex : 512 nm non filtrée - Emission après Ex : 512 nm filtrée) / (Emission après Ex : 490 nm non filtrée - Emission après Ex : 490 nm filtrée) ;
- utilisation de la courbe de calibration : pH intracellulaire = f (ratio 512/440 nm) pour déterminer le pH intracellulaire ;

Dans le cas de *Lactabacillus plantarum*, on a appliqué le même protocole de mesure avec les modifications suivantes : pH du tampon Phosphate de potassium 100 mM à pH 6,0 et température d'incubation 35°C au lieu de 30°C.

L'Homme de l'art adaptera le protocole aux spécificités de chaque souche bactérienne en fonction de la température et du pH optimaux de croissance.

Les compositions des solutions tampons sont à adapter par l'Homme du Métier pour chaque type de souche bactérienne testée, afin d'optimiser la mesure.

Le procédé de prédiction a été validé pour *Oenococcus oeni* par comparaison avec les paramètres de préparations non acclimatées et par la réalisation d'expériences de micro-vinification permettant de corréler les mesures effectuées avec la survie après implantation des bactéries dans le vin. Ainsi, les préparations acclimatées ont un fort niveau d'expression des gènes marqueurs et un pH intracellulaire significativement différent par rapport aux témoins, non acclimatés. Les expériences de micro-vinification ont mis en évidence une croissance de la population bactérienne pré-acclimatée, associée à une baisse de la concentration en acide malique, tandis que les populations non acclimatées survivaient très difficilement et n'avaient aucune incidence sur le taux d'acide malique.

Dans le cas du probiotique *Lactobacillus plantarum*, le procédé a été validé par corrélation avec la survie de bactéries acclimatées et non acclimatées qui ont été inoculées dans un milieu non nutritif à pH acide de 2,9, simulant les conditions acides du fluide gastrique de l'estomac.

On comprend bien l'avantage de l'utilisation de ce procédé de prédiction comme outil de contrôle qualité, qui permet d'obtenir très rapidement une indication du degré d'adaptation aux stress, et en particulier au stress acide, des préparations bactériennes, qu'elles soient obtenues par sélection ou par pré-acclimatation.

On notera que le procédé de prédiction employé est particulièrement robuste sur le plan statistique, les mesures effectuées et les résultats obtenus s'avérent précis et reproductibles.

Ce procédé permet, en outre, aux industriels d'ajuster bien plus efficacement leurs paramètres de pré-acclimatation et d'optimiser plus aisément leurs processus de pré-acclimatation. En effet, la mesure d'expression génique est réalisable en 48 heures à compter de la réception de la culture bactérienne, tandis que la mesure de pH intracellulaire nécessite 4 à 5 heures.

Le contrôle qualité de la stabilité de ces préparations bactériennes lors de leur stockage et/ou leur utilisation, en répétant dans le temps les mesures du procédé de prédiction, est donc également bien plus aisé et rapide par rapport aux techniques connues.

Il en va de même de l'utilisation du procédé selon l'invention pour la sélection de souches bactériennes, en particulier des ferments probiotiques.

Le procédé selon l'invention trouvera notamment une utilisation pour le contrôle qualité et/ou la sélection de préparations bactériennes dans les domaines de la nutrition humaine et animale (probiotiques), de l'agroalimentaire (processus fermentaires, en particulier du vin, de la bière, du maïs, du soja et dans les salaisons et charcuterie), de l'agriculture (inoculi bactériens) et de l'environnement (compostage, lutte contre les bactéries pathogènes et hygiénisation des effluents).

On utilisera également le procédé selon l'invention pour optimiser les procédés de production de ferments bactériens destinés à une utilisation dans des environnements stressants.

Enfin, il va de soi que le procédé selon l'invention peut être appliqué sans difficulté et sans effort excessif pour l'Homme du Métier à d'autres préparations bactériennes confrontées lors de leur utilisation à des environnements stressants et/ou hostiles, les marqueurs géniques étant bien entendu adaptés aux espèces considérées et les valeurs seuils du pHi étant déterminables sans difficultés particulières.

## Revendications

1. Procédé de prédiction de la viabilité, de la vitalité et de la stabilité de bactéries destinées à être utilisées dans des environnements stressants, lesquelles bactéries sont produites sous forme de préparations bactériennes et sont conditionnées sous forme congelée ou lyophilisée, **caractérisé en ce que** ledit procédé comporte :
- une étape préalable de remise en suspension desdites préparations bactériennes dans un milieu nutritif aqueux non stressant ;
- une mesure moléculaire du niveau d'expression d'au moins un gène marqueur représentatif de l'état de stress pour chaque espèce bactérienne considérée ;
- une mesure du pH intracellulaire des bactéries basée sur une sonde fluorescente sensible au pH ;
- une étape de comparaison des valeurs mesurées à celles de témoins et/ou d'étalons de référence.

2. Procédé selon la revendication 1 **caractérisé en ce que** la bactérie est *Oenococcus oeni* et **en ce que** la mesure moléculaire porte sur l'expression du gène *hsp18*.

3. Procédé selon la revendication 2 **caractérisé en ce que** la mesure moléculaire porte en outre sur l'expression d'au moins un gène pris parmi : *clpx, trxA, cfa*.

4. Procédé selon la revendication 1 **caractérisé en ce que** la bactérie est *Lactobacillus plantarum* et **en ce que** la mesure moléculaire porte sur l'expression du gène *hsp18.55*.

5. Procédé selon la revendication 4 **caractérisé en ce que** la mesure moléculaire porte en outre sur l'expression d'au moins un gène pris parmi : *hps19.5*, *groEL, cfa*.

6. Utilisation du procédé selon la revendication 1 pour le contrôle qualité de l'adaptation aux stress de ferments probiotiques.

7. Utilisation du procédé selon l'une quelconque des revendications 2 ou 3 pour le contrôle qualité de l'adaptation aux stress de ferments malolactiques destinés à l'ensemencement du vin.

8. Utilisation du procédé selon la revendication 1 pour la sélection de souches bactériennes utilisées pour la production de préparations bactériennes confrontées lors de leur utilisation à des environnements stressants.

## Claims

1. Method for predicting the viability, vitality and stability of bacteria, which are usable in stressing environments, produced in the form of bacterial preparations and packed in a frozen or lyophilised form, **characterised in that** said method comprises:
- a step consisting in pre-suspending said bacterial preparations in a non-stressing aqueous nutrient medium;
- molecular measurement of the level of expression of at least one marker gene representative of the stress state for each bacterial species in question;
- measurement of the intracellular pH of the bacteria based on a pH-sensitive fluorescent probe;
- a step consisting in comparing the values measured to those of references and/or reverence standards.

2. Method according to claim 1 **characterised in that** the bacterium is Oenococcus oeni and **in that** the molecular measurement relates to the expression of the hsp18 gene.

3. Method according to claim 2 **characterised in that** the molecular measurement also relates to the expression of at least one gene selected from: clpx, trxA, cfa.

4. Method according to claim 1 **characterised in that** the bacterium is Lactobacillus pantarum and **in that** the molecular measurement relates to the expression of the hsp18.55 gene.

5. Method according to claim 4 **characterised in that** the molecular measurement also relates to the expression of at least one gene selected from: hps19.5, groEL, cfa.

6. Use of the method according to claim 1 for controlling quality of stress adaptation of probiotic ferments.

7. Use of the method according to any of claims 2 or 3 for controlling quality of stress adaptation of malolactic ferments for inoculating wine.

8. Use of the method according to claim 1 for selecting bacterial strains used for the production of bacterial preparations subject to stressing environments during the use thereof.

## Patentansprüche

1. Verfahren zur Vorhersage der Lebensfähigkeit, der Vitalität und der Stabilität von Bakterien, die zur Verwendung in stressigen Umgebungen bestimmt sind, wobei diese Bakterien in Form von Bakterienpräparaten hergestellt und in gefrorener oder lyophilisierter Form aufbereitet sind, **dadurch gekennzeichnet, dass** das genannte Verfahren Folgendes umfasst:
- einen Vorabschritt des erneuten Suspendierens der genannten Bakterienpräparate in einem nicht stressigen, wässrigen Nährmedium;
- eine molekulare Messung des Grads der Expression mindestens eines Markergens, das für den Stresszustand repräsentatif ist, für jede betrachtete Bakterienspezies;
- eine Messung des intrazellulären pH-Werts von Bakterien, die auf einer für den pH-Wert empfindlichen Fluoreszenzsonde basiert;
- eignen Schritt des Vergleichs von Messwerten mit denen von Bezugssubstanzen und/oder -standards.

2. Verfahren nach Anspruch 1, **dadurch gekennzeichnet, dass** die Bakterie *Oenococcus oeni* ist und dass sich die molekulare Messung auf die Expression des Gens *hsp18* konzentriert.

3. Verfahren nach Anspruch 2, **dadurch gekennzeichnet, dass** sich die molekulare Messung außerdem auf die Expression mindestens eines Gens konzentriert, das aus folgenden ausgewählt ist: *clpx, trxA, cfa*.

4. Verfahren nach Anspruch 1, **dadurch gekennzeichnet, dass** die Bakterie *Lactobacillus plantarum* ist und dass sich die molekulare Messung auf die Expression des Gens *hsp18.55* konzentriert.

5. Verfahren nach Anspruch 4, **dadurch gekennzeichnet, dass** sich die molekulare Messung außerdem auf die Expression mindestens eines Gens konzentriert, das aus folgenden ausgewählt ist: *hsp19.5, groEL, cfa*.

6. Verwendung des Verfahrens nach Anspruch 1 zur Qualitätskontrolle der Stressanpassung von probiotischen Enzymen.

7. Verwendung des Verfahrens nach einem der Ansprüche 2 oder 3 zur Qualitätskontrolle der Stressanpassung von malolaktischen Enzymen, die zur Aussaat von Wein bestimmt sind.

8. Verwendung des Verfahrens nach Anspruch 1 zur Auswahl von Bakterienstämmen, die zur Herstellung von Bakterienpräparaten verwendet werden, die bei ihrer Verwendung stressigen Umgebungen ausgesetzt werden.
